# EUROPÄISCHE PATENTANMELDUNG

(11) **EP 3 273 152 A1**
(43) Veröffentlichungstag der Anmeldung: **24.01.2018**
(21) Anmeldenummer: 17184041.6
(22) Anmeldetag: 11.07.2016
(51) Int. Cl.: F21V 8/00, G01N 21/31, G01N 33/00, G01J 3/42

(54) **ANORDNUNG ZUM MESSEN VON GASKONZENTRATIONEN**

(62) Teilanmeldung aus: 16178808.8
(71) Anmelder: bluepoint medical GmbH & Co. KG, 23923 Selmsdorf (DE)
(72) Erfinder: LINDNER, Bernd, 23167 Stockelsdorf (DE)
(74) Vertreter: Seemann & Partner Patentanwälte mbB

(57) **Zusammenfassung**

Die Erfindung betrifft eine Anordnung zum Messen von Gaskonzentrationen in einem Gasabsorptionsverfahren, wobei die Anordnung mehrere Lichtquellen, eine Messzelle (3), wenigstens einen Messempfänger (5, 13, 14) und eine Auswertevorrichtung umfasst.

Erfindungsgemäß weist die Messzelle (3) einen längserstreckten schmalen Strahlengang mit einem eingangsseitigen Öffnungsdurchmesser *B* und einer Absorptionslänge *L* auf mit *L* > *B*, insbesondere *L* > *5·B*, insbesondere *L* > *10·B,* wobei die Messzelle (3) einen Gaseinlass (6) und einen Gasauslass (7) aufweist, wobei eine Mehrzahl von Lichtquellen unterschiedlicher Wellenlängenspektren zu einer ersten Lichtquellengruppe (10, 10') zusammengefasst ist, wobei ein optischer Homogenisator (11) zwischen die erste Lichtquellengruppe (10, 10') und die Messzelle (3) zwischengeschaltet ist, wobei insbesondere der Homogenisator (11) direkt oder über eine gemeinsame optische Baugruppe an die Lichtquellengruppe (10, 10') angekoppelt ist.

## Beschreibung

Die Erfindung betrifft eine Anordnung zum Messen von Gaskonzentrationen in einem Gasabsorptionsverfahren, in welchem Licht von Lichtquellen verschiedener Wellenlängen im sichtbaren, UV- und/oder IR-Bereich, insbesondere LED-Lichtquellen, durch eine Messzelle mit einer zu analysierenden Gasmischung geleitet wird, insbesondere einer Mischung von NO₂, SO₂ und/oder NO in Luft, und Gaskonzentrationen zu messender Gase der Gasmischung über eine Messung einer Abschwächung des in die Messzelle eingeleiteten Lichts bei verschiedenen Wellenlängen aufgrund von Absorption in den verschiedenen Gasen der Gasmischung bestimmt werden.

Die Messung von Gaskonzentrationen mittels eines Gasabsorptionsverfahrens ist ein bekanntes Verfahren der Messtechnik. Es beruht auf der Eigenschaft von Gasen, Licht bestimmter Wellenlängen zu absorbieren und somit abzuschwächen. Das Maß der Abschwächung ist dann ein Maß für die Gaskonzentration des absorbierenden Gases. Die Messung der Abschwächung der Lichttransmission durch das Gas erfordert in der Regel eine Referenzmessung, aus der entweder direkt oder indirekt auf die Intensität des in das Gas eingestrahlten Lichts geschlossen werden kann.

Bekannte Möglichkeiten einer Referenzmessung beinhalten beispielsweise die Abzweigung eines Anteils des zunächst ausgestrahlten Lichts, das dann auf einen optischen Messempfänger geleitet wird, ohne das Messgas zu durchlaufen. Bei dieser direkten Messung wird allerdings nicht berücksichtigt, dass weitere optische Elemente, wie beispielsweise Lichteintrittsfenster und Lichtaustrittsfenster einer Messzelle oder Lichtleiter selbst eine absorbierende Wirkung entfalten und beispielsweise durch Verschmutzung intransparenter werden können.

Eine alternative Möglichkeit hierzu bietet sich darin, Absorptionsbandlücken in dem Absorptionsspektrum des zu messenden Gases oder der zu messenden Gase zu benutzen, um Licht mit einer Wellenlänge durch die Messzelle zu leiten, von dem bekannt ist, dass es nicht oder nur wenig in dem Messgas oder den Messgasen absorbiert wird. Dieses Licht erleidet die gleiche Abschwächung in den übrigen optischen Elementen einer solchen Messanordnung wie dasjenige Licht, das beispielsweise auf die Absorptionsmaxima der Messgase eingestellt ist. Eine durch zunehmende Verschmutzung der Messzelle beispielsweise wird auf diese Weise ausgeschlossen. Beachtlich ist hierbei, dass die Wellenlängen der Referenzmessung nicht zu unterschiedlich von den Wellenlängen der eigentlichen Absorptionsmessung sein sollten, weil ansonsten dispersive Effekte, also wellenlängenabhängige Effekte, eine störende Rolle spielen können.

Eine Anordnung, mit der ein entsprechendes Gasabsorptionsverfahren durchführbar ist, ist in der Patentanmeldung DE 10 2008 064 173 A1 der Anmelderin beschrieben, deren Offenbarungsgehalt vollinhaltlich in die vorliegende Anmeldung aufgenommen sein soll. Die darin gezeigte Vorrichtung dient unter anderem zum Messen von Stickstoffmonoxid (NO), Stickstoffdioxid (NO₂), Schwefeldioxid (SO₂), Ozon (O₃) sowie Komponenten in fluiden Medien, für Verbrennungsmotoren, insbesondere bei der Online-Überwachung von Dieselmotoren, in der Umweltmesstechnik oder Medizintechnik, beispielsweise zur Messung der Atemluft. Darin werden verschiedenfarbige LEDs als Lichtquellen verwendet, deren Licht jeweils in eine Lichtleitfaser eingekoppelt wird.

Bei dieser Art der Lichterzeugung und Weiterleitung ist zu berücksichtigen, dass LEDs flächenhafte Lichterzeugungselemente sind und die Lichterzeugung nicht konstant über die gesamte Fläche der LEDs erfolgt. Vielmehr bilden sich zeitlich veränderliche Domänen auf den LEDs, die Licht emittieren, und die sich im Laufe der Zeit ändern, beispielsweise über die Oberfläche der LED wandern, sich teilen, erlöschen oder sich wieder vereinigen. Das Gleiche gilt für die Abstrahlungsrichtung, die zwar im Allgemeinen auf einen Abstrahlkegel innerhalb eines Abstrahlwinkels begrenzt ist, den Abstrahlkegel jedoch in zeitlich veränderlicher Weise bevölkert. Aus diesem Grund koppelt das von den LEDs erzeugte Licht in verschiedene Moden der Lichtleitfasern ein. Um zu verhindern, dass ausgangs der Lichtleitfasern Intensität und Abstrahlungsrichtung des aus den Lichtleitfasern austretenden Lichts fluktuiert, wird gemäß DE 10 2008 064 173 A1 eine Modenmischung vorgenommen, so dass das aus den Lichtleitfasern austretende Licht unabhängig geworden ist von dem ursprünglichen Ort und der ursprünglichen Richtung der Lichterzeugung.

Aus DE 10 2011 116 367 A1 ist eine alternative Anordnung bekannt, bei der eine sogenannte Multi Quantum Well-LED (MQW-LED) zum Einsatz kommt. Wie alle LEDs hat auch die MQW-LED ein Spektrum mit einer Breite von ca. 5 % bis 20 % der zentralen Wellenlänge, unterscheidet sich von herkömmlichen LEDs allerdings darin, dass die Form des Emissionsspektrums weitgehend temperaturunabhängig ist. Das Spektrum der MQW-LED wird gemäß DE 10 2011 116 367 A1 so gewählt, dass es bezüglich des Absorptionsspektrums eines zu messenden Gases Anteile hat, die eine starke Absorption erfahren, und andere Anteile, die eine geringe oder gar keine Absorption durch das zu messende Gas erfahren.

Ausgangs einer Messzelle wird das durchgestrahlte Licht der MQW-LED durch einen geeignet gewählten wellenlängenselektiven Strahlteiler in zwei Anteile aufgeteilt, bei denen in einem Referenzanteil wenig Absorption zu erwarten ist, und in einem anderen Anteil eine hohe Absorption entsprechend der Gaskonzentration des zu messenden Gases. Dies hat den Vorteil, dass aufgrund der engen Nachbarschaft der verschiedenen Frequenzanteile dispersive Effekte kaum auftreten und nur eine einzige Lichtquelle das Licht für die eigentliche Messung und für die Referenzmessung erzeugt. Voraussetzung hierfür ist die Temperaturstabilität des Emissionsspektrums, die bei herkömmlichen LEDs nicht gewährleistet ist. Auch die Offenbarung von DE 10 2011 116 367 A1 soll vollumfänglich in die vorliegende Patentanmeldung aufgenommen sein.

Demgegenüber liegt der vorliegenden Erfindung die Aufgabe zugrunde, eine kompakte Anordnung zur Gasabsorptionsmessung zur Verfügung zu stellen, mit der Gaskonzentrationen mehrerer Gase eines Gasgemisches auch unter extremen Umweltbedingungen mit hoher Genauigkeit und Geschwindigkeit messbar sind.

Diese Aufgabe wird durch eine Anordnung zum Messen von Gaskonzentrationen in einem Gasabsorptionsverfahren gemäß Patentanspruch 1 gelöst.

In einem erfindungsgemäßen Gasabsorptionsverfahren wird Licht von Lichtquellen verschiedener Wellenlängen im sichtbaren, UV- und/oder IR-Bereich, insbesondere LED-Lichtquellen, durch eine Messzelle mit einer zu analysierenden Gasmischung geleitet, insbesondere einer Mischung von NO₂, SO₂ und/oder NO in Luft, und werden Gaskonzentrationen zu messender Gase der Gasmischung über eine Messung einer Abschwächung des in die Messzelle eingeleiteten Lichts bei verschiedenen Wellenlängen aufgrund von Absorption in den verschiedenen Gasen der Gasmischung bestimmt. Die erfindungsgemäße Anordnung umfasst mehrere Lichtquellen, deren unterschiedliche Wellenlängenspektren auf Absorptionsbänder, Absorptionslücken und/oder Übergangsbereiche zwischen Absorptionsbändern und Absorptionslücken der zu messenden Gase abgestimmt sind, eine Messzelle, wenigstens einen Messempfänger, mittels dessen eine Lichtintensität bei einer oder mehreren der eingestrahlten Wellenlängen ausgangs der Messzelle messbar ist, und eine Auswertevorrichtung, die ausgebildet ist, aus den gemessenen Lichtintensitäten die Gaskonzentrationen zu bestimmen, umfasst.

Diese Anordnung ist erfindungsgemäß dadurch weitergebildet, dass die Messzelle einen längserstreckten schmalen Strahlengang mit einem eingangsseitigen Öffnungsdurchmesser *B* und einer Absorptionslänge *L* aufweist mit *L > B*, insbesondere *L >* 5·*B*, insbesondere *L > 10·B*, wobei die Messzelle einen Gaseinlass und einen Gasauslass aufweist, wobei eine Mehrzahl von Lichtquellen unterschiedlicher Wellenlängenspektren zu einer ersten Lichtquellengruppe zusammengefasst ist, wobei ein optischer Homogenisator zwischen die erste Lichtquellengruppe und die Messzelle zwischengeschaltet ist, wobei insbesondere der Homogenisator direkt oder über eine gemeinsame optische Baugruppe an die Lichtquellengruppe angekoppelt ist.

Unter einer direkten Ankopplung wird im Rahmen der vorliegenden Erfindung eine Ankopplung mit einem Luftspalt oder einem optischen Medium verstanden, beispielsweise einem optischen Kleber, gegebenenfalls auch mehreren optischen Klebepunkten. Eine geeignete gemeinsame optische Baugruppe kann beispielsweise eine Sammellinse oder Linsengruppe sein, die das Licht aller Lichtquellen der Lichtquellengruppe gemeinsam empfängt und auf den Homogenisator leitet. Auch ein Hohlspiegel oder ein vor der Lichtquellengruppe angeordnetes Multi-Linsen-Array ist ein solches geeignetes gemeinsames optisches Element. Nicht dazu gehören allerdings beispielsweise individuelle Lichtleitfasern, die jeweils nur an eine Lichtquelle der Lichtquellengruppe angekoppelt werden.

Der optische Homogenisator ist insbesondere ein Lichtleiter oder ist als solcher ausgebildet. Ferner insbesondere ist der optische Homogenisator stabförmig ausgebildet. Schließlich ist insbesondere vorgesehen, dass der optische Homogenisator ein stabförmiger Lichtleiter ist oder als solcher ausgebildet ist.

Die Erfindung beruht auf dem Grundgedanken, dass eine schnelle Messung schnell veränderlicher Gaskonzentrationen nur in sehr kleinen Messvolumina möglich ist, deren Gasinhalt auch sehr schnell ausgetauscht wird. Ansonsten würde ein langsamer Austausch des Messgases in der Messzelle dazu führen, dass schnelle Änderungen der Gaskonzentrationen ausgewaschen werden. Für eine hohe Genauigkeit ist allerdings prinzipbedingt eine große Absorptionslänge notwendig. Dies ist einerseits durch eine große Länge der Messzelle selbst und andererseits durch ein- oder mehrfache Spiegelung in der Messzelle realisierbar. Um gleichzeitig das Messvolumen klein zu halten, ist eine schmale bzw. schlanke längserstreckte Form des Strahlengangs in der Messzelle erfindungsgemäß gewählt worden. Die Form der Messzelle sollte an den Strahlengang möglichst gut angepasst sein, um so wenig wie möglich Totvolumen außerhalb des Strahlengangs aufzuweisen.

Ein weiterer entscheidender Faktor für eine hohe Messgenauigkeit liegt darin, dass eine möglichst große Lichtmenge durch die Messzelle zu einem Empfänger gebracht werden muss. Dabei ist es bei einer längserstreckten schmalen Messzelle eine Herausforderung, das von der Lichtquelle ausgesandte Licht überhaupt größtenteils in die Messzelle hinein zu bekommen. Um dies zu gewährleisten, wird zunächst die Mehrzahl der Lichtquellen, insbesondere LEDs, zu einer sehr kleinen und kompakten Lichtquellengruppe zusammengefasst. Diese bildet eine "mehrfarbige" kleine Lichtquelle. Die einzelnen Lichtquellen der Lichtquellengruppe sollten möglichst dicht beieinander angeordnet sein, um möglichst wenig Totfläche zwischen ihnen zu haben und somit ein hohes Verhältnis aus leuchtender zu nichtleuchtender Fläche zu haben.

Die Emissionsfläche der Lichtquellengruppe und der Abstrahlwinkel der Lichtquellengruppe definieren den zur Verfügung stehenden Phasenraum des ausgesandten Lichts, der möglichst vollständig in die Messzelle eingetragen werden sollte, um eine hohe Messgenauigkeit zu gewährleisten. Es ist zu beachten, dass der Strahlengang in der Messzelle einen sehr kleinen Phasenraum aufspannt. Die Lichtquellengruppe sollte dementsprechend eine kleine Gesamtemissionsfläche aufweisen, weil auf diese Weise von Beginn an nur ein kleiner Phasenraum bevölkert bzw. erzeugt wird. Ein Verlust an Lichtleistung und eine gewisse Selektion und Verkleinerung des Phasenraums erfolgt im Homogenisator, der hierdurch den vom durchgelassenen Licht eingenommenen Phasenraum an den Phasenraum des Strahlengangs der Messzelle anpasst, wobei gegebenenfalls noch optische Baugruppen aus Linsen oder Spiegel zum Einsatz kommen können. Das zur Verfügung stehende ursprünglich emittierte Licht wird auf diese Weise mit nur vergleichsweise geringen Verlusten in die Messzelle mit kleinem Strahlengangdurchmesser eingekoppelt wird. Der kleine Durchmesser ist somit förderlich für ein geringes Messvolumen und eine hohe zeitliche Auflösung der Messung, wobei gleichzeitig die Ausgangsleistung der Lichtquellen vergleichsweise gering gehalten werden kann.

Aufgrund der Zusammenstellung mehrerer einzelner Lichtquellen zu einer Lichtquellengruppe handelt es sich hierbei um einen nichtzusammenhängenden Phasenraum, so dass jeder Einschnitt durch Aperturen oder sonstige Hindernisse die Zusammenstellung und Verteilung der verschiedenen Wellenlängenspektren stark zulasten der Intensität einzelner Wellenlängen bzw. Lichtquellen beeinflussen kann. Da Gasabsorptionsmessungen bei Gasgemischen auch die Messungen der relativen Stärken des transportierten Lichts verschiedener Farben bzw. Wellenlängen beinhalten, würde eine solche selektive Beeinflussung die Ergebnisse verfälschen. Aus diesem Grund ist erfindungsgemäß ein Homogenisator vorhanden, der dafür sorgt, dass der zur Verfügung stehende Phasenraum, also die räumliche Verteilung und Winkelverteilung der einzelnen Lichtstrahlen, möglichst gleichmäßig von allen eingestrahlten Wellenlängen bevölkert wird, so dass unvermeidbare Einschnitte nicht selektiv zulasten einzelner Wellenlängen gehen. Dafür sammelt der Homogenisator das Licht der verschiedenen LEDs der Lichtquellengruppe, mischt ihre Herkunftsorte gleichmäßig und strahlt das Licht gleichmäßig und mit möglichst kleiner Apertur bzw. Eingangsöffnung ab, wobei die Lichtverluste der Übertragung von den Quellen zur Zielapertur bzw. Zielöffnung möglichst gering sein sollte. Solche Bauteile sind klein, robust und kostengünstig herzustellen und erlauben eine Miniaturisierung gegenüber den bisher verwendeten Glasfasern, die außerdem noch Faserkoppler und Modenmischer benötigen. Gegebenenfalls durch den Homogenisator erzeugte Intensitätsverluste sind gegenüber dem zuvor genannten Vorteil in Kauf zu nehmen.

Die Merkmalskombination des schlanken bzw. schmalen Strahlengangs in der Messzelle zusammen mit der auf einem kleinen Raum beziehungsweise einer kleinen Oberfläche zusammengefassten mehrfarbigen Lichtquellengruppe und dem dazwischen geschalteten Homogenisator erlaubt es nunmehr erstmals, die hohe Auflösung und Genauigkeit eines Gasabsorptionsmessverfahrens mit einer für eine kurze Messzeit notwendigen Miniaturisierung zu verknüpfen, ohne Kompromisse bei der Messgenauigkeit eingehen zu müssen.

Der Durchmesser der Lichtquellengruppe kann kleiner sein als eine Fläche des Öffnungsdurchmessers *B* des Strahlengangs der Messzelle, insbesondere weniger als ein Drittel der Fläche der Öffnung betragen. Der Homogenisator kann vorteilhafterweise ferner an der Seite der ersten Lichtquellengruppe eine Fläche aufweisen, die einer Fläche der ersten Lichtquellengruppe im Wesentlichen entspricht, insbesondere nicht mehr als 40% von der Fläche der ersten Lichtquellengruppe abweicht. Diese Ausführung erlaubt eine besonders starke Miniaturisierung, da der Homogenisator mit den entsprechenden Größenverhältnissen direkt auf die Lichtquellengruppe aufgesetzt werden kann und das ausgestrahlte Licht zu einem großen Teil weiterleiten kann. Eine etwas weniger miniaturisierte Ausführungsform wird erreicht, wenn die Eintrittsfläche des Homogenisators deutlich größer als die Fläche der ersten Lichtquellengruppe ist, insbesondere mehr als doppelt so groß. Diese Ausführungsform ist dann vorteilhaft, wenn baubedingt eine Beabstandung zwischen Lichtquellengruppe und Homogenisator notwendig ist.

Ein ausgangsseitiger Durchmesser *D*₂ des Homogenisators an der Seite der Messzelle ist vorzugsweise kleiner als der Öffnungsdurchmesser *B* des Strahlengangs der Messzelle.

Bei der Auswertevorrichtung handelt es sich um eine programmierbare Datenverarbeitungsanlage mit Signaleingängen und Signalverarbeitungseinheiten oder um einen digitalen Signalprozessor (DSP) oder Mikrocontroller mit vorgeschalteten Analog-Digitalwandlern oder um andere zu Messzwecken üblicherweise verwendete Analysatoren.

Mit der erfindungsgemäßen Anordnung sind Messgenauigkeiten im ppm-Bereich und kleiner mit Messwiederholungsraten von 10/s bis 100/s erzielbar.

In einer vorteilhaften Weiterbildung umfasst die erste Lichtquellengruppe LED-Lichtquellen mit einem charakteristischen Abstrahlwinkel und ist die LED-Lichtquellengruppe so vor dem Homogenisator angeordnet, dass ein Abstrahlkegel der LED-Lichtquellen der ersten Lichtquellengruppe, gegebenenfalls nach Durchtritt durch die gemeinsame optische Baugruppe, im Wesentlichen vollständig in den Homogenisator eintritt. Der Abstrahlkegel umfasst einen Großteil des ausgestrahlten Lichts und umfasst nach einer Definition den Winkel, der von den seitlichen Punkten mit halber Maximal-Lichtstärke eingeschlossen wird. Mit dieser Maßnahme wird sichergestellt, dass Intensitätsverluste beim Eintritt in den Homogenisator so gering wie möglich gehalten werden. Dies trägt dazu bei, eine hohe Messgenauigkeit zu erreichen.

Vorzugsweise ist der Homogenisator als ein geformter transparenter massiver Lichtleiter auf der Grundlage von Totalreflexion an der Oberfläche oder von Brechzahlgradienten im Substrat oder als hohle Reflektoranordnung mit einem transparenten Medium im Inneren, insbesondere einem transparenten Gas oder Vakuum, und verspiegelten seitlichen Begrenzungsflächen ausgebildet, wobei der Homogenisator linear oder gebogen mit kreisrundem, ovalem oder polygonalem Querschnitt ausgebildet ist. Insbesondere ist der Homogenisator im Querschnitt sechseckig. Durch Mehrfachreflexion oder -beugung bevölkert sich der zur Verfügung stehende Phasenraum in diesen Homogenisatoren zunehmend gleichmäßig. Eine weitere Verdichtung der Belegung des Phasenraumes ergibt sich, wenn sich vorteilhafterweise der Homogenisator im Querschnitt in Richtung zur Messzelle hin verändert, insbesondere verjüngt.

Die Homogenisierung wird vorzugsweise weiter dadurch verbessert, dass Störstellen im oder am Homogenisator angeordnet sind, insbesondere Fehlstellen im Substrat, Streukörper in einem Spiegelhohlraum oder Rauigkeiten an Grenzflächen oder Spiegelflächen. Solche Störstellen führen zu einer starken Streuung des Lichts. Die dadurch erreichte starke Homogenisierung wird mit einem höheren Verlust an Lichtintensität durch Streuung aus dem Homogenisator heraus erkauft, jedoch kann der Vorteil der deutlich stärkeren Homogenisierung des einfallenden Lichts den Verlust an zur Verfügung stehender Lichtintensität mehr als wettmachen.

Die Messzelle weist an ihrer Eingangsöffnung und/oder ein an die Messzellen angeordnetes Element der Anordnung weist ein kombiniertes Lichteintritts- und -austrittsfenster und eine gegenüber dem Lichteintritts- und -austrittsfenster liegende lichtreflektierende Wand auf oder, alternativ, jeweils ein Lichteintrittsfenster und ein Lichtaustrittsfenster, mit oder ohne spiegelnde Wände zwischen dem Lichteintrittsfenster und dem Lichtaustrittsfenster, wobei das oder die Lichteintrittsfenster und/oder Lichtaustrittsfenster insbesondere gegenüber einer Längserstreckung der Messzelle schräg gestellt ist oder sind. Der Fall des kombinierten Lichteintritts- und -austrittsfensters mit der lichtreflektierenden, also spiegelnden, Wand beschreibt den Fall einer Reflexionszelle, der andere Fall den einer Transmissionszelle.

Der Abschluss der Messzelle durch Fenster ermöglicht eine Abtrennung der Messzelle vom restlichen optischen Messaufbau und somit deren Austausch, Reinigung, Wartung und Ersatz. Eine Schrägstellung der Fenster verringert störende Reflexionen und ist mechanisch günstig zum Einfügen in einen Aufbau. Ebenfalls werden strömungstechnische Totvolumina etwas verringert. Die Messzelle kann auch als Ganzes aus dem Aufbau herausgenommen werden, sodass ein einfacher Austausch gegebenenfalls zum Zwecke der Wartung oder Reinigung möglich ist.

Gemäß einer weiteren Ausführungsform ist daher vorgesehen, dass die Messzelle herausnehmbar ist. Insbesondere ist die Messzelle werkzeuglos demontierbar.

Zu Kalibrierzwecken kann die Messzelle vorteilhaft durch eine Kalibrierzelle mit definierter wellenlängenabhängiger Absorption ersetzt werden, was teure Kalibriergase einspart. Falls das oder die Fenster nicht an der Messzelle selbst, sondern an einem angrenzenden Element der Anordnung angeordnet sind, besteht die Messzelle aus einer Röhre, die mittels einer Dichtung gasdicht gegenüber dem Fenster oder den Fenstern eingesetzt wird. Diese Variante hat den Vorteil, dass die Fenster gleich in der Anordnung gereinigt werden können und die restliche Anordnung vor Reinigungsflüssigkeit schützen. Es ergibt sich hierdurch eine modulare, insbesondere hermetische, Anordnung.

Vorzugsweise sind der Gaseinlass und der Gasauslass parallel zum Durchmesser der Messzelle versetzt angeordnet, um einen raschen Gasaustausch zu ermöglichen.

Wenn vorteilhafterweise eingangs und/oder ausgangs der Messzelle ein oder mehrere insbesondere wellenlängenselektive Strahlteiler angeordnet ist oder sind, mit dem oder mit denen Licht unterschiedlicher Lichtquellen der ersten Lichtquellengruppe und/oder einer zweiten Lichtquellengruppe zu zwei oder mehr verschiedenen Messempfängern geleitet wird, ist es möglich, die verschiedenen Wellenlängen, die die verschiedenen Lichtquellen der Lichtquellengruppe ausgestrahlt haben, und die auf Absorptionsbänder und Absorptionslücken verschiedener Messgase ausgelegt sind, wieder voneinander zu trennen und unabhängig voneinander die Abschwächung bei diesen Wellenlängen zu messen.

Die der Erfindung zugrunde liegende Aufgabe wird auch durch eine Anordnung zum Messen von Gaskonzentrationen in einem Gasabsorptionsverfahren, in welchem Licht von Lichtquellen verschiedener Wellenlängen im sichtbaren, UV- und/oder IR-Bereich, insbesondere LED-Lichtquellen, durch eine Messzelle mit einer zu analysierenden Gasmischung geleitet wird, insbesondere einer Mischung von NO₂, SO₂ und/oder NO in Luft, und Gaskonzentrationen zu messender Gase der Gasmischung über eine Messung einer Abschwächung des in die Messzelle eingeleiteten Lichts bei verschiedenen Wellenlängen aufgrund von Absorption in den verschiedenen Gasen der Gasmischung bestimmt werden, wobei die Anordnung mehrere Lichtquellen mit unterschiedlichen Wellenlängenspektren, eine Messzelle und mehrere Messempfänger umfasst, mittels deren Lichtintensitäten bei mehreren der eingestrahlten Wellenlängen ausgangs der Messzelle messbar sind, gelöst, die dadurch weitergebildet ist, dass die Messzelle an ihren beiden Enden jeweils Öffnungen aufweist und eine erste Lichtquellengruppe und eine zweite Lichtquellengruppe mit jeweils einer Lichtquelle oder mehreren zusammen gruppierten Lichtquellen umfasst sind, deren Licht auf zwei voneinander unabhängigen Strahlwegen, insbesondere in zueinander entgegengesetzten Richtungen, durch die Messzelle geleitet wird, und das aus der Messzelle austretende Licht der beiden Strahlwege jeweils durch insbesondere wellenlängenselektive Strahlteiler ausgangs der Messzelle zu entsprechenden Messempfängern geleitet wird, wobei wenigstens eine der Lichtquellengruppen zusammen mit dem dazu gehörenden Strahlweg und der Messzelle als zuvor beschriebene erfindungsgemäße Anordnung ausgebildet ist.

Diese erfindungsgemäße Anordnung umfasst die zuvor beschriebene erfindungsgemäße Anordnung vollständig und führt ihr noch einen weiteren Strahlweg und eine weitere Lichtquelle hinzu. Diese zweite Lichtquellengruppe kann eine einzelne Lichtquelle umfassen, womit der Anordnung eine weitere Wellenlänge für ein weiteres Messgas der Gasmischung hinzugefügt wird. Die zweite Lichtquellengruppe kann auch mehrere unterschiedliche Lichtquellen umfassen.

Grundsätzlich hat die erfindungsgemäße Anordnung den Vorteil, dass mehrere Gase in ein und demselben Messvolumen gemessen werden können, was die Messgenauigkeit erhöht, da räumliche und dynamische Fehler bei der Gasaufteilung auf mehrere Messzellen vermieden werden. Vorzugsweise überlappen hierzu die beiden Strahlwege in einem Messvolumen der Messzelle teilweise oder ganz.

Für die Messung mehrerer Gase mittels LED-Spektroskopie werden mehrere Lichtquellen benötigt, die aus unterschiedlichen Richtungen in die Messzelle angekoppelt werden können, um so ein zeitlich paralleles Messen mit geringem Übersprechen zu ermöglichen. Dies bietet sich besonders auch bei der Kombination unterschiedlicher Messverfahren an, wie sie beispielsweise aus DE 10 2008 064 173 A1 und DE 10 2011 116 367 A1 bekannt sind. Anstelle der teuren und großen Glasfasern, die in DE 10 2008 064 173 A1 verwendet werden, kommt eine Miniaturisierung zum Einsatz, da die benötigten LEDs sehr eng nebeneinander positioniert werden und das emittierte Licht nur von einem Homogenisator aufgefangen und homogenisiert wird und dann über einen externen Strahlteiler, beispielsweise einen halbdurchlässigen Spiegel, in einen Pfad zur Gasmessung und einen Referenzpfad aufgeteilt wird.

Für den Fall, dass die beiden Strahlwege gegenläufig durch die Messzelle führen, sind die Messungen mit dem ersten Strahlweg und dem zweiten Strahlweg ideal entkoppelt, was besonders vorteilhaft ist, wenn die verwendeten Wellenlängen der Lichtquellen nahe beieinander liegen. Es gibt aber auch andere Möglichkeiten, die Strahlwege voneinander im Wesentlichen zu entkoppeln, beispielsweise die Definition verschiedener Bereiche der Messzelle, die von den einzelnen Strahlwegen bestrahlt werden oder von Winkeln, unter denen die Strahlwege in die Messzelle eintreten.

In einer bevorzugten Weiterbildung sind beide Lichtquellengruppen zusammen mit dem jeweils dazu gehörenden Strahlweg und der Messzelle als zuvor beschriebene erfindungsgemäße Anordnungen ausgebildet, wobei die Messzelle beiden Anordnungen gemeinsam ist. Alternativ und ebenfalls bevorzugt umfasst eine bzw. die zweite Lichtquellengruppe wenigstens eine MQW-LED mit temperaturstabilem Emissionsspektrum und der zur zweiten Lichtquellengruppe gehörende Strahlweg ausgangs der Messzelle einen wellenlängenselektiven Strahlteiler und zwei Messempfänger, wobei der wellenlängenselektive Strahlteiler eingerichtet ist, das Emissionsspektrum der MQW-LED in zwei oder mehr Anteile aufzuspalten und die Anteile getrennt voneinander zu den beiden Messempfängern zu leiten.

Im letzteren Fall entspricht die Teilmessanordnung mit der MQW-LED im Wesentlichen derjenigen, die aus DE 10 2011 116 367 A1 der Anmelderin bekannt ist. Diese Version ist besonders gut für Gase mit schmalbandigen Absorptionsspektren geeignet wie beispielsweise Stickstoffmonoxid (NO). Solche Filter können Kantenfilter oder Bandpassfilter sein.

Im Kontext der vorliegenden Beschreibung wird unter einem "wellenlängenselektiven Strahlteiler" sowohl ein einzelner Strahlteiler, der die Strahlteilung wellenlängenselektiv vornimmt, als auch eine Gruppe von optischen Bauelementen verstanden. Eine solche Gruppe von optischen Bauelementen umfasst beispielsweise einen Strahlteiler, welcher nicht wellenlängenselektiv ist, und zumindest zwei Filter. In den geteilten Strahlengängen ist in Lichteinfallsrichtung auf den Strahlteiler folgend jeweils einer der Filter vorgesehen. Bei den Filtern handelt es sich beispielsweise um Kanten- oder Bandpassfilter, welche die Funktionalität der Wellenlängenselektion bereitstellen.

Das Emissionsspektrum der wenigstens einen MQW-LED sowie eine Wellenlängencharakteristik des wellenlängenselektiven Strahlteilers sind vorzugsweise so auf ein Absorptionsspektrum eines zu messenden Gases abgestimmt, dass ein erster Anteil des Emissionsspektrums der MQW-LED eine höhere Absorption im Gas erfährt als ein zweiter Anteil. Damit ist das Licht der einen MQW-LED in einen Messanteil und einen Referenzanteil aufgespalten. Systematische Effekte werden so auf ideale Weise unterdrückt. Auch, wenn der Referenzanteil nicht vollständig ohne Absorption ist, so sind doch diese Emissionsspektren der MQW-LED und die Gasabsorptionsspektren der zu messenden Gase bekannt, sodass durch die Messung im Messanteil und im Referenzanteil auf den Fall der idealen Nichtabsorption extrapoliert werden kann, woraus sich die zu bestimmende Gaskonzentration dann ergibt.

Wenn vorzugsweise die Anordnung eine Druck- und/oder Temperaturmesseinrichtung umfasst, die mit der Messzelle zur Messung eines Drucks und/oder einer Temperatur des Gasgemisches in der Messzelle verbunden ist, wobei die Auswertevorrichtung ausgebildet ist, den Einfluss eines gemessenen Druckniveaus oder von Druckschwankungen und/oder der Temperatur oder Temperaturschwankungen auf die Lichtabsorption oder die Gaskonzentrationen bei der Bestimmung der Gaskonzentrationen zu berücksichtigen, gegebenenfalls auf einen Normaldruck und/oder eine Normaltemperatur zu extrapolieren, dann ist es möglich, solche Umwelteinflüsse bei der Bestimmung der Gaskonzentrationen zu berücksichtigen, die einen eigenen Einfluss auf die Lichtabschwächung haben. Diese sind in besonderem Maße der Druck des Gasgemisches und die Temperatur.

In einer vorteilhaften Ausgestaltung wird Messgas in einem Nebenstromverfahren aus einem Hauptgasstrom in die Messzelle geleitet und nach Durchlaufen der Messzelle wieder in den Hauptgasstrom eingeleitet oder in die Umgebungsluft entlassen. Dieses Nebenstromverfahren erlaubt es, die Anordnung in einem größeren Abstand von einer für die Lichtquellen und Messempfänger unzuträglichen Umgebung anzuordnen. Dies ist beispielsweise in den Motoren von Kraftfahrzeugen, insbesondere Dieselmotoren, der Fall.

Falls eine weitere Beabstandung der Lichtquellen oder der Messempfänger von der Messzelle gewünscht ist, erfolgt vorzugsweise eine Lichteinkopplung in die Messzelle und/oder eine Lichtauskopplung aus der Messzelle unter Verwendung zusätzlicher Lichtleiter. Falls das Messvolumen direkt in einer unzuträglichen Umgebung liegen soll, ist eine zusätzliche Erhöhung des Abstandes der Messempfänger, Lichtquellen und Elektroniken von der Messzelle gewünscht. Hierzu erfolgt vorzugsweise die Lichteinkopplung in die Messzelle und/oder die Lichtauskopplung aus der Messzelle unter Verwendung zusätzlicher Lichtleiter.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1a), b): Emissions- und Absorptionsspektren von Messgasen und LEDs
- Fig. 2: eine schematische Präsentation einer erfindungsgemäßen Anordnung,
- Fig. 3: Beispiele erfindungsgemäßer Homogenisatoren,
- Fig. 4: eine schematische Darstellung eines Teils einer erfindungsgemäßen Anordnung,
- Fig. 5: eine schematische Darstellung einer Homogenisierung ohne Streuzentren,
- Fig. 6: eine schematische Darstellung einer Homogenisierung mit Streuzentren,
- Fig. 7a)-d): schematische Darstellungen von Lichtleitungsprinzipien erfindungsgemäßer Homogenisatoren und
- Fig. 8: das spektrale Überlappungs- und Aufteilungsprinzip bei der Verwendung von MQW-LED ist zur Gasabsorptionsmessung.

In den Zeichnungen sind jeweils gleiche oder gleichartige Elemente und/oder Teile mit denselben Bezugsziffern versehen, so dass von einer erneuten Vorstellung jeweils abgesehen wird.

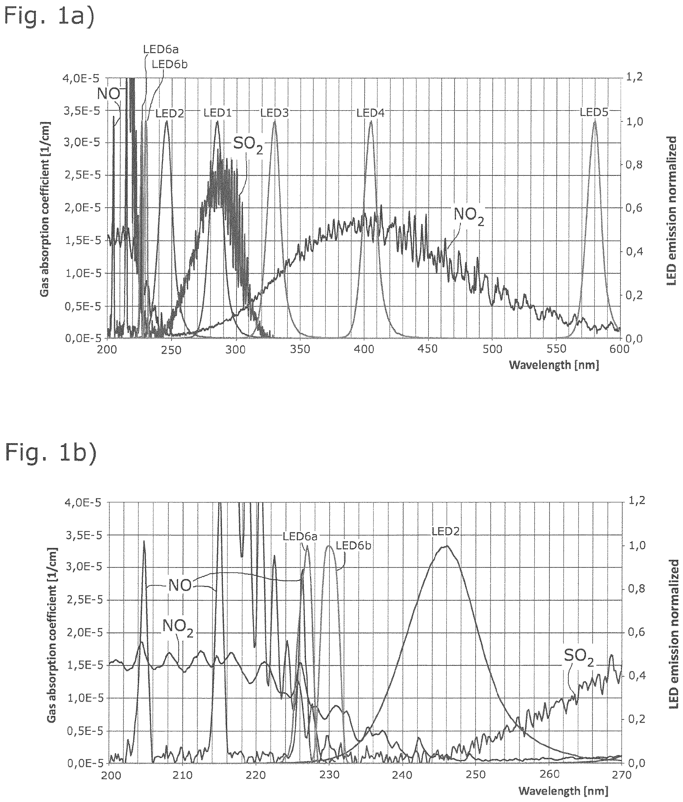

In den Figuren 1a) und 1b) sind jeweils die Absorptionsspektren der Gase Stickstoffmonoxid (NO), Schwefeldioxid (SO₂) und Stickstoffdioxid (NO₂) dargestellt, zusammen mit den Emissionsspektren von sechs verschiedenen LEDs, die willkürlich auf eine gemeinsame Maximalemission normalisiert sind. Ein solches Gasgemisch kommt beispielsweise im Abgas von Kraftfahrzeugmotoren vor, wobei die Verhältnisse von NO und NO₂ von der Temperatur abhängen. Die Wellenlängen beginnen bei 200 nm, also im UV-Bereich, und gehen in Figur 1a) bis 600 nm. Der Ausschnitt in Figur 1b) befindet sich vollständig im UV-Bereich zwischen 200 nm und 270 nm.

Das Absorptionsspektrum von NO₂ ist besonders breitbandig und hat ein Maximum bei ca. 400 nm. Sowohl die LED3 bei 330 nm als auch die LED4 bei ca. 405 nm erfahren eine signifikante Absorption in NO₂. Die längerwellige LED5 bei 580 nm kann als Referenz dienen, da sie sehr viel weniger Absorption in NO₂ erfährt. Im UV-Bereich befindet sich das schmalere Absetzungsspektrum von SO₂ mit einer Breite von ca. 60 nm um das Maximum bei 285 nm. Die LED1 ist mit ihrem Emissionsspektrum hierauf zentriert. Eine weitere LED2 ist mit ihrem Emissionsspektrum um ein Maximum bei ca. 246 nm zentriert und liegt in einem lokalen Minimum der Absorptionsspektren aller drei dargestellter Gase. Damit eignet sich diese Wellenlänge der LED 2 als Referenzwellenlänge und hat den weiteren Vorteil beispielsweise gegenüber LED5, dass neben einer geringen Absorption auch die dispersiven Effekte gegenüber den Wellenlängen von beispielsweise LED1 und LED3, auch LED 4, geringer sind.

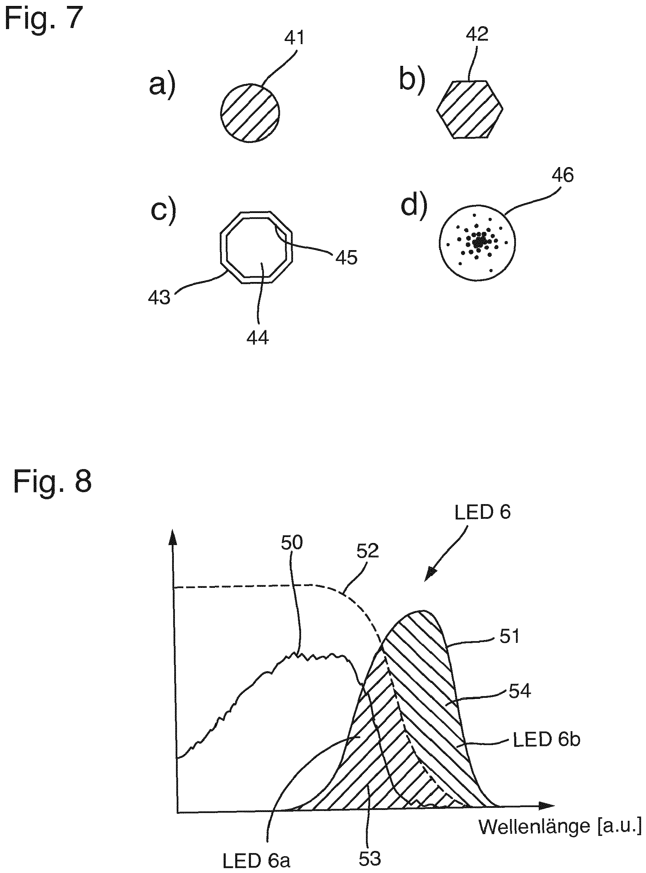

Wie besonders gut in Figur 1b) zu erkennen ist, besteht das Absorptionsspektrum von NO aus mehreren schmalen Bändern mit Breiten von wenigen Nanometern bei 205 nm, 215 nm und 226 nm. Eine MQW-LED mit der Bezeichnung LED6 erzeugt Licht in einem Bereich mit einer Breite von ca. 8 nm um 248 nm herum. Das Spektrum ist aufgeteilt in zwei Bereiche mit den Bezeichnungen LED6a und LED6b, die durch Aufspaltung in einem wellenlängenselektiven interferometrischen Strahlteiler mit steiler Flanke erzeugt werden. Dabei hat der kürzerwellige Anteil LED6a einen großen Überlapp mit dem Absorptionspeak von NO bei 226 nm, während der längerwellige Anteil LED6b kaum einen Überlapp mit dem Absorptionspeak hat und somit als Referenz aus derselben Lichtquelle verwendet werden kann. Das Prinzip ist unten in Figur 8 noch einmal deutlicher erläutert.

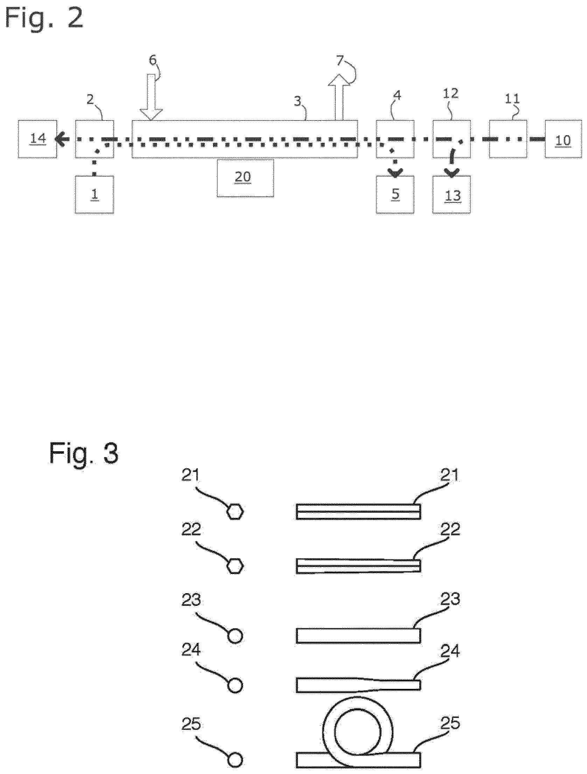

Figur 2 zeigt eine schematische Darstellung einer erfindungsgemäßen Anordnung, in deren Zentrum eine Messzelle 3 mit einem Messgas bzw. Gasgemisch steht. Bei der Messzelle 3 handelt es sich um eine schlanke, langgestreckte, beispielsweise zylindrische Messzelle 3, was den Vorteil hat, dass bei geringem Volumen und somit möglicher hoher Austauschrate des Messgases eine große Absorptionslänge erreichbar ist. Hierfür verfügt die Messzelle 3 über einen Gaseinlass 6 und einen Gasauslass 7.

Auf der rechten Seite ist eine Lichtquellengruppe 10 dargestellt, in der mehrere verschiedenfarbige Lichtquellen, beispielsweise LEDs, erfindungsgemäß auf kleinem Raum zusammengefasst sind. Deren ausgestrahltes Licht gelangt durch einen Homogenisator 11, der im Folgenden noch näher erläutert wird, und über Strahlteiler 12, 4 zur Messzelle 3. Der Strahlteiler 12 kann die Funktion erfüllen, einen Teil des eingestrahlten Lichts als Referenz zu einem Referenz-Messempfänger 13 zu leiten. Dazu kann der Strahlteiler 12 wellenlängenselektiv sein, muss es aber nicht. Der restliche Anteil des Lichts aus der Lichtquellengruppe 10 tritt durch die gesamte Länge der Messzelle 3 hindurch, erfährt dabei eine wellenlängenabhängige Absorption im Gasgemisch und gelangt am anderen Ende zum Messempfänger 14, der ausgebildet ist, die Intensität des auf ihn fallenden Lichts zu messen. Durch Vergleich mit einem Sollwert oder durch Vergleich mit der Intensität im Referenz-Messempfänger 13 wird dann die Abschwächung berechnet oder ermittelt und daraus die ansprechenden Gaskonzentrationen der zu untersuchenden Gase bestimmt.

Mit der Messzelle 3 ist eine Druck- und/oder Temperaturmesseinrichtung 20 verbunden, die Druck und oder Temperatur des Gases in der Messzelle 3 misst und an die nicht dargestellte Auswertevorrichtung weiterleitet, die hieraus Korrekturen an der Bestimmung der Gaskonzentrationen vornehmen kann.

Die Erfindung gemäß Anordnung gemäß Figur 2 weist einen zweiten Messpfad auf, der dem ersten, zuvor beschriebenen Messpfad entgegengesetzt ausgerichtet ist. Der zweite Meßpfad beginnt mit einer Lichtquellengruppe 1, die im einfachsten Fall eine einzelne im MQW-LED umfasst oder aufweist. Es können auch mehrere MQW-LEDs umfasst sein oder Mischungen verschiedener LEDs mit oder ohne MQW-LED. In dem gezeigten Ausführungsbeispiel ist wenigstens eine MQW-LED umfasst, beispielsweise die LED6 der Figur 1. Ein Strahlteiler 2 lenkt das Licht der Lichtquellengruppe 1, also der MQW-LED, in die Messzelle 3 ein und trennt die Strahlwege des ersten Messpfads und des zweiten Messpfads voneinander. Nach Durchlaufen der Messzelle trennt ein Strahlteiler 4, der ein wellenlängenselektiver Strahlteiler sein kann, wiederum die beiden Messpfade bzw. Strahlwege voneinander und lenkt das Licht aus der Lichtquellengruppe 1 in eine Messanordnung mit einem Messempfänger 5. Diese Messanordnung kann einen weiteren Strahlteiler umfassen, der eine Aufteilung mit einer steilen Flanke entsprechend der obigen Beschreibung und der Figur 8 hierunter vornimmt. In diesem Fall sind zwei optische Messempfänger, beispielsweise Fotodioden, im Einsatz.

Anstelle zweier Strahlteiler 4, 12 kann auch ein gemeinsamer Strahlteiler verwendet werden, der entsprechend ausgebildet ist, beide Strahlwege wellenlängenselektiv voneinander zu entkoppeln, wobei er für die Wellenlängen der ersten Lichtquellengruppe 10 teiltransparent und teilreflektierend sein sollte. Die Messempfänger 5 und 13 sind in einem solchen Fall an gegenüberliegenden Seiten des Hauptstrahlengangs angeordnet.

In Figur 3 sind verschiedene Beispiele von erfindungsgemäßen Homogenisatoren 21 bis 25 schematisch dargestellt. Der Homogenisator 21, der auf der linken Seite im Querschnitt und auf der rechten Seite von der Seite dargestellt ist, ist ein herkömmlicher massiver Lichtleiter mit 6-eckigen bzw. hexagonalem Querschnitt. Der Homogenisator 22 unterscheidet sich hiervon darin, dass er sich vom Eingang zum Ausgang hin verjüngt, was zu einer stärkeren Homogenisierung beiträgt. Solche hexagonale Querschnitte haben die Eigenschaft, dass an den Stoßkanten zwischen 2 glatten Flächen vermehrt Lichtverluste auftreten, während sie bei runden Querschnitten stärker über den gesamten Umfang verteilt sind.

Der Homogenisator 23 hat im Gegensatz zu dem Homogenisator 21 einen runden Querschnitt. Der Homogenisator 24 hat ebenfalls einen runden Querschnitt, verjüngt sich allerdings. Der Homogenisator 24 schließlich hat einen runden Querschnitt und einen konstanten Durchmesser, beschreibt allerdings eine Biegung um 360°, was zu einer starken Homogenisierung führt. In allen Fällen handelt es sich um massive Homogenisatoren, beispielsweise aus Glas oder Plexiglas.

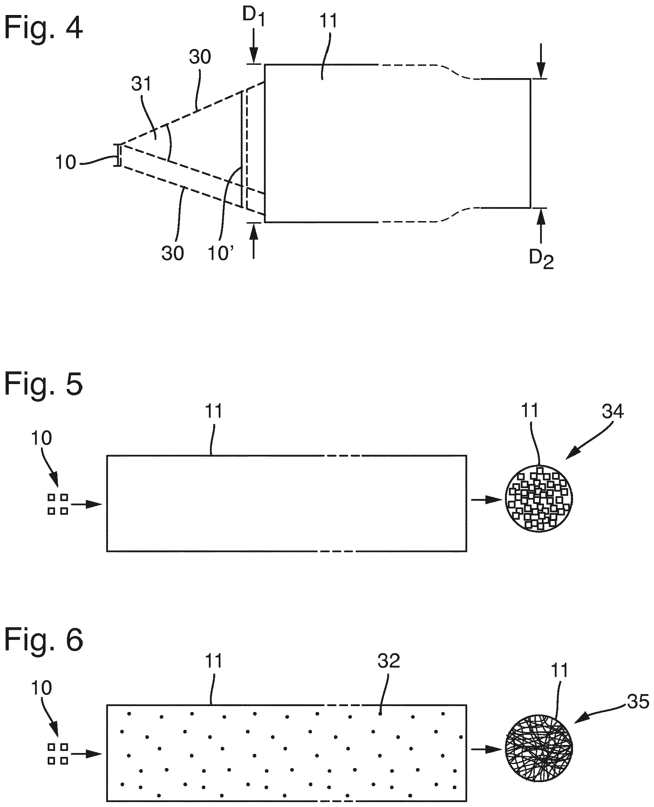

In Figur 4 ist schematisch die Kombination aus Lichtquellengruppe 10 und Homogenisator 11 dargestellt. Die Lichtquellengruppe 10 verfügt über mehrere als kurze Striche angedeutete Einzel-LEDs verschiedener Wellenlängen bzw. Farben, die auf einem kleinen Raum aneinander gesetzt sind. Dabei wird es sich um einen Durchmesser dieser Lichtquellengruppe 10 von üblicherweise weniger als 1 bis 2 mm handeln. Wie die meisten LEDs haben diese LEDs einen Abstrahlwinkel 31, was zu einem vollständigen Abstrahlkegel 30 der Lichtquellengruppe 10 führt, der von der Gesamtfläche der Lichtquellengruppe 10 ausgeht und sich zur Eintrittsfläche des Homogenisators 11 hin ausweitet. Der Abstand zwischen der Lichtquellengruppe 10 und der Eingangsfläche des Homogenisators 11 ist so gewählt, dass der Kegel 30 bei Eintritt in den Homogenisator 11 einen Durchmesser aufweist, der kleiner ist als die Öffnungsapertur *D₁* des Homogenisators 11. Im Inneren des Homogenisators, der sich zum Ausgang hin zu einem kleineren Durchmesser *D₂* verjüngt, findet eine Lichtleitung mit keinem oder wenigem Intensitätsverlust statt.

Den gleichen Abstrahlkegel 30 spannt auch eine im Verhältnis größere Lichtquellengruppe 10' auf, die umso näher am Homogenisator 11 angeordnet ist. Zum Zwecke stärkerer Miniaturisierung ist die Wahl günstiger, den Homogenisator 11 in etwa so groß zu machen wie die Lichtquellengruppe 10' und ihn entsprechend dicht vor die Lichtquellengruppe 10' zu platzieren.

In den Figuren 5 und 6 ist jeweils dargestellt, wie der Phasenraum, in diesem Fall die Belegung der räumlichen Verteilung, durch den jeweiligen Homogenisator 11 vergleichmäßigt wird. Die räumliche Verteilung des ausgestrahlten Lichtes ist zunächst die räumliche Anordnung der LEDs der Lichtquellengruppe 10, in den Figuren 5 und 6 jeweils links dargestellt. Nach Durchgang durch den Homogenisator, der in Figur 5 ohne Störstellen und in Figur 6 mit Störstellen 32 versehen ist, ergibt sich im ersten Fall eine vielfache Spiegelung sämtlicher LEDs, die im Wesentlichen gleichmäßig die Austrittsfläche des Homogenisators 11 bedecken. Die einzelnen LEDs sind allerdings als solche noch im Wesentlichen erkennbar. Wenn, wie in Figur 6, Störstellen 32 anzutreten, erzeugt die zusätzliche Streuung des Lichts im Homogenisator 11 ein vollständiges Verwaschen, so dass die einzelnen Lichtquellen nicht mehr erkennbar sind. Dies geht allerdings auf Kosten eines Intensitätsverlustes durch aus dem Homogenisator 11 herausgestreutes Licht. Anstelle von Störstellen 32 kann auch die äußere Oberfläche aufgebaut sein, sodass sich Störstellen in der Totalreflexion ergeben, die einen ähnlichen Effekt haben wie eingebettete Störstellen 32.

In Figur 7a) bis d) sind wiederum Querschnitte verschiedener Ausführungsformen erfindungsgemäß einsetzbarer Homogenisatoren gezeigt. Der Homogenisator 40 gemäß Figur 7a) hat einen runden Querschnitt und ist massiv. Die Lichtleitung erfolgt in diesem Fall durch Totalreflexion an der Außenfläche. Davon unterscheidet sich der Homogenisator 42 aus Figur 7 b) lediglich in der Form, die in diesem Fall hexagonal ist. Hier konzentrieren sich Lichtverluste auf die Kanten zwischen den einzelnen Seitenflächen.

Figur 7c) zeigt ein Beispiel eines als Reflektoranordnung ausgebildeten Homogenisators 43, der sich in seinen Außenkonturen nicht von derjenigen aus Figur 7b) unterscheidet. Innen ist er jedoch hohl und weist um seinen Innenraum 44 herum verspiegelte Innenflächen 45 auf. Figur 7d) schließlich betrifft wiederum einen massiven Homogenisator 46, der im Unterschied zu dem Ausführungsbeispiel der Figur 7a) im Zentrum einen höheren Brechungsindex aufweist als am Rand, sodass sich eine Lichtleitung durch Beugung aufgrund des Brechungsindexgradienten ergibt. An der Grenzfläche zur Umgebungsluft findet auch eine Totalreflexion statt.

In Figur 8 ist das Prinzip der Streckenaufteilung bei MQW-LED noch einmal schematisch verdeutlicht. Wiederum ist auf der horizontalen Achse eine Wellenlänge in willkürlichen Einheiten aufgetragen, auf der vertikalen Achse jeweils eine Absorptions-, Transmission- oder eine Emissionsamplitude in willkürlichen Einheiten angegeben. Mit dem Bezugszeichen 50 ist das Absorptionsspektrum eines Messgases mit einer vergleichsweise steilen Flanke bezeichnet. Das Emissionsspektrum 51 einer MQW-LED, beispielsweise der LED6 aus Figur 1, ist so gewählt, dass es nur teilweise mit der abfallenden Flanke des Absetzungsspektrums 50 überlappt. Entsprechend ist mit einer gestrichelten Linie 52 eine Wellenlängencharakteristik eines interferometrischen Strahlteilers mit steiler Flanke eingezeichnet, dessen Flanke im Wesentlichen das Emissionsspektrum 51 in zwei Teile aufteilt, die zu unterschiedlichen Messempfängern, beispielsweise Fotodioden, weitergeleitet werden. Diese beiden Anteile sind unterschiedlich schraffiert und mit dem Bezugszeichen 53 und 54 für einen Signalanteil und einen Referenzanteil bezeichnet. Der Signalanteil 43 hat einen starken Überlapp mit der abfallenden Flanke des Absorptionsspektrum 50, während der Referenzanteil 54 kaum einen Überlapp damit hat. Diese Anteile 53 und 54 sind in Figur 1a) und Figur 1b) als LED6a und LED6b bezeichnet und getrennt voneinander dargestellt, auch wenn sie aus einer einzigen Lichtquelle stammen. Statt des gezeigten Kurzpassfilters kann auch ein Bandpassfilter eingesetzt werden.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 1: Lichtquellengruppe mit MQW-LED
- 2: spektraler Strahlteiler
- 3: Messzelle
- 4: spektraler Strahlteiler
- 5: Messempfänger
- 6: Gaseinlass
- 7: Gasauslass
- 10, 10': Lichtquellengruppe
- 11: Homogenisator
- 12: Strahlteiler
- 13: Referenz-Messempfänger
- 14: Messempfänger
- 20: Druck- und/oder Temperaturmesseinrichtung
- 21-25: Homogenisator
- 30: Abstrahlkegel
- 31: Abstrahlwinkel
- 32: Störstellen
- 34, 35: Intensitätsverteilung ausgangs des Homogenisators
- 41: homogener runder Homogenisator
- 42: homogener hexagonaler Homogenisator
- 43: innenverspiegelter Homogenisator
- 44: Hohlraum
- 45: Innenverspiegelung
- 46: Homogenisator mit Brechungsindexgradient
- 50: Absorptionsspektrum eines Gases
- 51: Emissionsspektrum einer MWQ-LED
- 52: Wellenlängencharakteristik eines interferometrischen Strahlteilers
- 53: Signalanteil
- 54: Referenzanteil

## Patentansprüche

1. Anordnung zum Messen von Gaskonzentrationen in einem Gasabsorptionsverfahren, in welchem Licht von Lichtquellen verschiedener Wellenlängen im sichtbaren, UV- und/oder IR-Bereich, insbesondere LED-Lichtquellen, durch eine Messzelle (3) mit einer zu analysierenden Gasmischung geleitet wird, insbesondere einer Mischung von NO₂, SO₂ und/oder NO in Luft, und Gaskonzentrationen zu messender Gase der Gasmischung über eine Messung einer Abschwächung des in die Messzelle (3) eingeleiteten Lichts bei verschiedenen Wellenlängen aufgrund von Absorption in den verschiedenen Gasen der Gasmischung bestimmt werden, wobei die Anordnung mehrere Lichtquellen, deren unterschiedliche Wellenlängenspektren auf Absorptionsbänder, Absorptionslücken und/oder Übergangsbereiche zwischen Absorptionsbändern und Absorptionslücken der zu messenden Gase abgestimmt sind, eine Messzelle (3), wenigstens einen Messempfänger (5, 13, 14), mittels dessen eine Lichtintensität bei einer oder mehreren der eingestrahlten Wellenlängen ausgangs der Messzelle (13) messbar ist, und eine Auswertevorrichtung, die ausgebildet ist, aus den gemessenen Lichtintensitäten die Gaskonzentrationen zu bestimmen, umfasst, **dadurch gekennzeichnet, dass** die Messzelle (3) einen längserstreckten schmalen Strahlengang mit einem eingangsseitigen Öffnungsdurchmesser *B* und einer Absorptionslänge *L* aufweist mit *L > B*, insbesondere *L > 5*·*B*, insbesondere *L* > *10*·*B*, wobei die Messzelle (3) einen Gaseinlass (6) und einen Gasauslass (7) aufweist, wobei eine Mehrzahl von Lichtquellen unterschiedlicher Wellenlängenspektren zu einer ersten Lichtquellengruppe (10, 10') zusammengefasst ist, wobei ein optischer Homogenisator (11) zwischen die erste Lichtquellengruppe (10, 10') und die Messzelle (3) zwischengeschaltet ist, wobei insbesondere der Homogenisator (11) direkt oder über eine gemeinsame optische Baugruppe an die Lichtquellengruppe (10, 10') angekoppelt ist.

2. Anordnung nach Anspruch 1, **dadurch gekennzeichnet, dass** die erste Lichtquellengruppe (10, 10') LED-Lichtquellen mit einem charakteristischen Abstrahlwinkel (31) umfasst, und die LED-Lichtquellengruppe (10, 10') so vor dem Homogenisator (11) angeordnet ist, dass ein Abstrahlkegel (30) der LED-Lichtquellen der ersten Lichtquellengruppe (10, 10'), gegebenenfalls nach Durchtritt durch die gemeinsame optische Baugruppe, im Wesentlichen vollständig in den Homogenisator (11) eintritt.

3. Anordnung nach Anspruch 1 oder 2, **dadurch gekennzeichnet, dass** der Homogenisator (11) als ein geformter transparenter massiver Lichtleiter (41, 42, 46) auf der Grundlage von Totalreflexion an der Oberfläche oder von Brechzahlgradienten im Substrat oder als hohle Reflektoranordnung (43) mit einem transparenten Medium im Inneren, insbesondere einem transparenten Gas oder Vakuum, und verspiegelten seitlichen Begrenzungsflächen (45) ausgebildet ist, wobei der Homogenisator (11) linear oder gebogen mit kreisrundem, ovalem oder polygonalem Querschnitt ausgebildet ist.

4. Anordnung nach Anspruch 3, **dadurch gekennzeichnet, dass** der Homogenisator (11) sich im Querschnitt in Richtung zur Messzelle (3) hin verändert, insbesondere verjüngt.

5. Anordnung nach einem der Ansprüche 1 bis 4, **dadurch gekennzeichnet, dass** Störstellen (32) im oder am Homogenisator (11) angeordnet sind, insbesondere Fehlstellen im Substrat, Streukörper in einem Spiegelhohlraum oder Rauigkeiten an Grenzflächen oder Spiegelflächen.

6. Anordnung nach einem der Ansprüche 1 bis 5, **dadurch gekennzeichnet, dass** die Messzelle (3) an ihrer Eingangsöffnung und/oder ein an die Messzelle (3) angrenzendes Element der Anordnung ein kombiniertes Lichteintritts- und -austrittsfenster und eine gegenüber dem Lichteintritts- und -austrittsfenster liegende lichtreflektierende Wand auf oder, alternativ, jeweils ein Lichteintrittsfenster und ein Lichtaustrittsfenster, mit oder ohne spiegelnde Wände zwischen dem Lichteintrittsfenster und dem Lichtaustrittsfenster, wobei das oder die Lichteintrittsfenster und/oder Lichtaustrittsfenster insbesondere gegenüber einer Längserstreckung der Messzelle (3) schräg gestellt ist oder sind.

7. Anordnung nach einem der Ansprüche 1 bis 6, **dadurch gekennzeichnet, dass** eingangs und/oder ausgangs der Messzelle (3) ein oder mehrere insbesondere wellenlängenselektive Strahlteiler (2, 4) angeordnet ist oder sind, mit dem oder mit denen Licht unterschiedlicher Lichtquellen der ersten Lichtquellengruppe (10, 10') und/oder einer zweiten Lichtquellengruppe (1) zu zwei oder mehr verschiedenen Messempfängern (5, 14) geleitet wird.

8. Anordnung nach einem der Ansprüche 1 bis 7, **dadurch gekennzeichnet, dass** die Anordnung eine Druck- und/oder Temperaturmesseinrichtung (20) umfasst, die mit der Messzelle (3) zur Messung eines Drucks und/oder einer Temperatur des Gasgemischs in der Messzelle (3) verbunden ist, wobei die Auswertevorrichtung ausgebildet ist, den Einfluss eines gemessenen Druckniveaus oder von Druckschwankungen und/oder der Temperatur oder Temperaturschwankungen auf die Lichtabsorption oder die Gaskonzentrationen bei der Bestimmung der Gaskonzentrationen zu berücksichtigen, gegebenenfalls auf einen Normaldruck und/oder eine Normaltemperatur zu extrapolieren.

9. Anordnung nach einem der Ansprüche 1 bis 8, **dadurch gekennzeichnet, dass** eine Lichteinkopplung in die Messzelle (3) und/oder eine Lichtauskopplung aus der Messzelle (3) unter Verwendung zusätzlicher Lichtleiter erfolgt.

10. Anordnung nach einem der Ansprüche 1 bis 9, **dadurch gekennzeichnet, dass** die Messzelle (3) herausnehmbar ist.
